# EUROPEAN PATENT APPLICATION

(11) **EP 3 654 049 A1**
(43) Date of publication of application: **20.05.2020**
(21) Application number: 18206551.6
(22) Date of filing: 15.11.2018
(51) Int. Cl.: G01R 33/28, A61B 5/055

(54) **EDDY CURRENT BRAKE FOR PATIENT TABLE OF MRI**

(71) Applicant: Koninklijke Philips N.V., 5656 AG Eindhoven (NL)
(72) Inventor: Weiss, Steffen, 5656 AE Eindhoven (NL); Johnson, Mark Thomas, 5656 AE Eindhoven (NL); Helle, Michael Günter, 5656 AE Eindhoven (NL); Djajadiningrat, Johan Partomo, 5656 AE Eindhoven (NL); Vogtmeier, Gereon, 5656 AE Eindhoven (NL)
(74) Representative: de Haan, Poul Erik

(57) **Abstract**

The present invention relates to a patient support (10). In order to improve safety for MRI scanning protocols, a patient support is provided for an MRI scanner. The patient support comprises a braking device (12) for deaccelerating the patient support when being transferred relative to the MRI scanner. The braking device comprises at least one non-magnetic electrically conductive element. The at least one non-magnetic electrically conductive element is configured to induce one or more eddy currents in response to a magnetic field of the MRI scanner to provide a counter force against an attractive force between the patient support and the MRI scanner, thereby creating a braking effect.

## Description

### FIELD OF THE INVENTION

The present invention relates to a patient support. In particular, the present invention relates to a patient support for a magnetic resonance imaging scanner and a magnetic resonance imaging system.

### BACKGROUND OF THE INVENTION

The workflow of diagnostic imaging exams with a magnetic resonance imaging (MRI) system includes the transfer of patients in and out of the scanner on a patient support table. It is known that the presence of any magnetic material will cause a strong attractive force to move the material into the bore of the MRI. This may result in an accidental collision of the patient support table.

CN 107019604 A describes a mobile hospital bed with a bed frame, a hand push frame and universal rollers. The universal rollers are provided with electromagnetic brakes, which are controlled by a healthcare professional via capacitive touch keys arranged on the hand push frame.

### SUMMARY OF THE INVENTION

There may be a need to improve the safety for MRI scanning protocols.

The object of the present invention is solved by the subject-matter of the independent claims, wherein further embodiments are incorporated in the dependent claims. It should be noted that the following described aspects of the invention apply also for the patient support and for the MRI system.

A first aspect of the invention relates to a patient support for an MRI scanner. The patient support comprises a braking device for deaccelerating the patient support when being transferred relative to the MRI scanner. The braking device comprises at least one non-magnetic electrically conductive element. The at least one non-magnetic electrically conductive element is configured to induce one or more eddy currents in response to a magnetic field of the MRI scanner to provide a counter force against an attractive force between the patient support and the MRI scanner, thereby creating a braking effect.

In other words, a patient support, such as a patient bed or transport unit, may be equipped with one or more non-magnetic electrically conductive elements, e.g. below a patient matrice, which use induced eddy currents to deaccelerate the patient support when moving towards an MRI scanner. The braking effect may be achieved by the interaction of the non-magnetic metal material on the patient support and the external magnetic field of an MRI scanner; that is, the braking effect may be realized passively or automatically in response to the magnetic field of the MRI scanner without any inputs, e.g. button, from a person. This may advantageously provide an additional safety feature for regular MRI scanning protocols. It may be of particular advantage where entirely or partly autonomous scanning is considered and a healthcare professional may not always be present to avoid accidental collisions of the patient support, as will be explained hereafter in more detail.

The "patient support" as used herein may be e.g. a bed, a transport unit, a scan table.

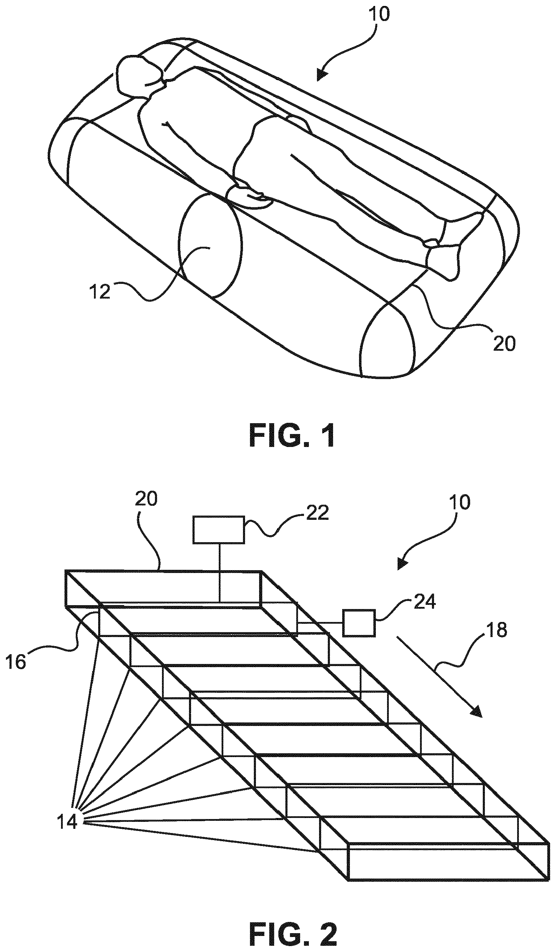

The "non-magnetic electrically conductive elements" as used herein may comprise metal elements and/or closed loops of conductive wire. The non-magnetic electrically conductive elements may comprise Aluminium, Copper or any other suitable materials to create the desired eddy currents. The non-magnetic electrically conductive elements may be arranged and configured differently to realize adjustable eddy currents in response to the changeable magnetic fields of the MRI. For example, the volume of metal elements and/or closed loops of conductive wire may increase over a length of the patient support. The closed loops of conductive wire interruptible by switches may be used to avoid large masses and to be able to switch off the eddy-current brake by a software or a user, e.g. when the patient support moves out of the bore. The metal elements may be brought together for increased eddy current creation, optionally with e.g. small magnetic components or by breakage of stoppers to realize automatic movement, and/or optionally with an actuators to actively move the metal elements apart for decreased eddy current creation. These arrangements and configurations will be explained in more detail hereafter and particularly with respect to the exemplary embodiments of Figs. 2 to 8.

The "attractive force", or "magnetic attractive force", as used herein may be present during a transfer of the patient table towards a gradient of magnetic field. The attractive force is not constant; it varies during a transfer of the patient support towards the MRI scanner and is strongest in the region of the flanges of the bore.

As will become apparent from the present disclosure, the function of the "eddy current" is to provide a braking force as the patient support enters the MRI bore, for example. This may slow down the patient support during entry into the bore and makes it easier for the autonomous system to safely position the patient in the bore without unwanted positions. Furthermore, in the case that some magnetic material is accidentally present, the eddy current brake will oppose the magnetic attractive force and again reduce the impact of collision.

According to an embodiment of the invention, the braking device comprises a plurality of non-magnetic electrically conductive elements. The plurality of non-magnetic electrically conductive elements is configured and arranged to adjust the induced eddy currents in response to the magnetic field such that the counter force is adjustable against the attractive force during a transfer of the patient support relative to the MRI scanner.

In other words, the braking device may comprise two or more non-magnetic electrically conductive elements. These elements may be arranged on the patient support in a particular way to adjust the eddy currents. These elements may also be configured in a particular way (e.g. with electrically controllable elements and/or mechanically moveable elements) to adjust the eddy currents. The various arrangements and configurations of the plurality of non-magnetic electrically conductive elements will be explained in more detail hereafter and particularly with respect to the exemplary embodiments of Figs. 2 to 8.

During operation, which can be autonomous, the amount of braking force that will be required by the system is not always constant. The braking force may vary depending upon the position of the patient support in the bore or the amount of magnetic material accidentally present in the patient support. Thus, it may be advantageous to adjust the arrangement and configuration of the non-magnetic electrically conductive elements during a transfer of the patient support relative to the MRI scanner.

According to an embodiment of the invention, the at least one non-magnetic electrically conductive elements comprises a closed loop of conductive wire.

The closed loops of conductive wire may also be referred to as electrically controllable elements, which may comprise Aluminium, Copper or any other suitable materials.

The closed loops of conductive wire may advantageously introduce less masses compared to e.g. a non-magnetic metal block into the patient support. Moreover, any interference with the radiofrequency coils (RF coils) on the patient support may be avoided in the off-state. The configurations and associated advantages of the closed loops of conductive wire will be explained in more detail in the text of the exemplary embodiments of Figs. 2 and 6-8.

According to an embodiment of the invention, at least one of the closed loops of conductive wire is provided with a switch configured for switching the eddy currents on and off. The switch comprises at least one of the following: a software controlled switch, and a user controlled switch.

A user may control the user controlled switch either directly, for example, via a device wired to the patient support, or indirectly via a software, such as wireless remote control, apps, etc.

Advantageously, the braking effect may be switched off when the patient support is moved out of the bore.

According to an embodiment of the invention, at least one of the closed loops of conductive wire is configured to have low loop impedance in a passive state such that in an event of power outage the braking effect is present. The loop impedance may be changed by introducing at least one element into the loop the impedance of which can be controlled externally between low and high impedance. The use of several elements in one loop is advantageous, because any interference with the RF coil or gradient coils with the loop in the patient support is be avoided if all elements are in the off-state.

In this context, low impedance shall be understood that this element allows a relatively large amount of current through, per unit of applied voltage at that point. Typical low impedance values are below 1Ohm.

According to an embodiment of the invention, the at least one non-magnetic electrically conductive element comprises a non-magnetic metal block.

The non-magnetic metal blocks may be referred to as mechanically moveable elements, which may comprise Aluminium, Copper, or any other suitable materials. The non-magnetic metal blocks may have various designs, such as a set of non-magnetic metal blocks without any interruption of the area of the conducting material, non-magnetic metal blocks with slots, non-magnetic metal blocks in form of a comb and a rectangular block, or non-magnetic metal blocks in form of two interdigitated combs.

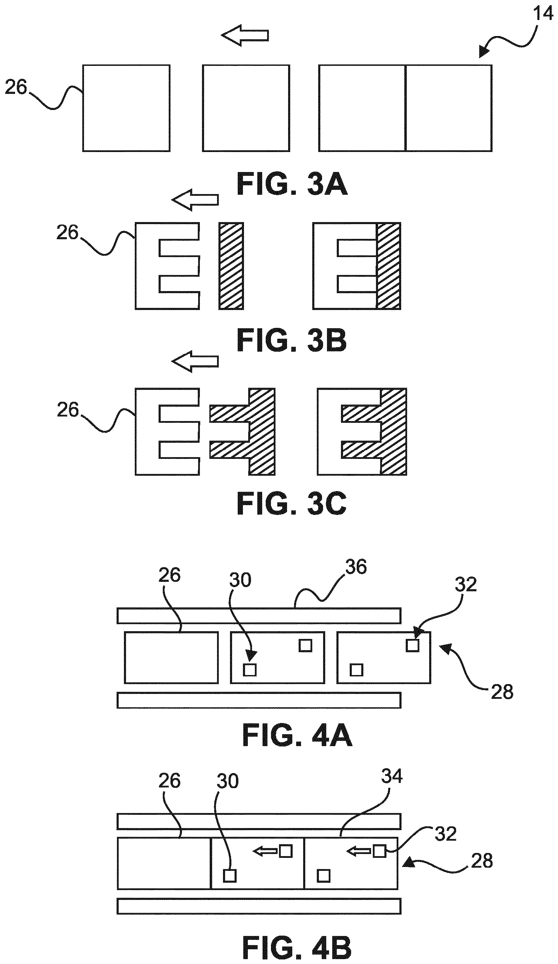

Various arrangements and configurations of the non-magnetic metal blocks will be explained in more detail in the text of the exemplary embodiments of Figs. 3 to 8.

According to an embodiment of the invention, each non-magnetic metal block has a cross sectional area perpendicular to a primary magnetic field direction of the magnetic field. The non-magnetic metal blocks are provided with an element joining device configured for moving the non-magnetic metal blocks from electrically isolated positions to electrically contacting positions to increase the cross sectional area perpendicular to the primary magnetic field direction during a transfer of the patent support towards the magnetic field of the MRI scanner, thereby increasing the breaking effect. Alternatively or additionally, the non-magnetic metal blocks are provided with an element separating device configured for moving the non-magnetic metal blocks from electrically contacting positions to electrically isolated positions to decrease the cross sectional area perpendicular to the primary magnetic field direction during a transfer of the patient support away from the magnetic field of the MRI scanner, thereby decreasing the breaking effect.

The amount of braking force created by a given volume of conductive material is not a constant, but may be modified by adjusting the dimensions of the volume. For example, a block of Aluminium with a single large area can provide a stronger eddy current braking force than the same volume divided into a set of smaller blocks or any interruption of the area of the conducting area. This embodiment may enable the use of conductive eddy current brakes where it is possible to introduce gaps (or separations) into the area of the surface of the non-magnetic metal blocks where the eddy currents are circulating (i.e. the area perpendicular to the primary magnetic field direction or BO-field direction) for decreased eddy current creation. The non-magnetic metal blocks may be brought together for increased eddy current creation. In a first option, a series of solid non-magnetic metal blocks (i.e. non-magnetic metal blocks with no interruption of the area) may be brought together for increased eddy current direction, as will be explained in more detail in the text of the exemplary embodiments of Fig. 3A. In a second option, a set of non-magnetic metal blocks with slots may be brought together by closing one or more of the slots for increased eddy current creation, as will be explained in more detail in the text of the exemplary embodiments of Figs. 3B and 3C.

In electrically isolated positions, the non-magnetic metal blocks are kept separate in electrically non-contacting positions. Thus, the eddy currents are circulating over a separate (or isolated) area of each non-magnetic metal block, respectively. In electrically contacting positions, the non-magnetic metal blocks are joined together so that the eddy currents can circulate over a larger area of the joined non-magnetic metal blocks and the braking force increases.

The element joining device may thus advantageously bring the non-magnetic metal blocks together for increased eddy current creation, thereby increasing the braking force. The element separating device may advantageously separate the joint non-magnetic metal blocks apart for decreased eddy current creation, thereby decreasing the braking force. In addition, braking force is only increased when the cross-section perpendicular to the primary magnetic field direction increases. Thus, it may be advantageous to increase/decrease the cross-section perpendicular to the primary magnetic field direction to adjust the braking force, as will be explained in more detail in the text of the exemplary embodiments of Figs. 4 to 5.

According to an embodiment of the invention, the element joining device comprises a plurality of magnetic components, each arranged on a respective non-magnetic metal block. Each magnetic component has a dimension that is large enough to cause the attached non-magnetic metal block to move. Alternatively or additionally, the element joining device comprises a guiding mechanism along the length of the patient support. The guiding mechanism comprises a plurality of stoppers along the guiding mechanism for keeping the non-magnetic metal blocks in electrically isolated positions. The plurality of stoppers is configured to allow the non-magnetic metal blocks to move from electrically isolated positions to electrically contacting positions under the guidance of the guiding mechanism if the attractive force exceeds a certain measure. According to an embodiment of the invention, the element separating device comprises at least one actuator.

The dimension of the magnetic component may be small enough not to cause the patient support to move.

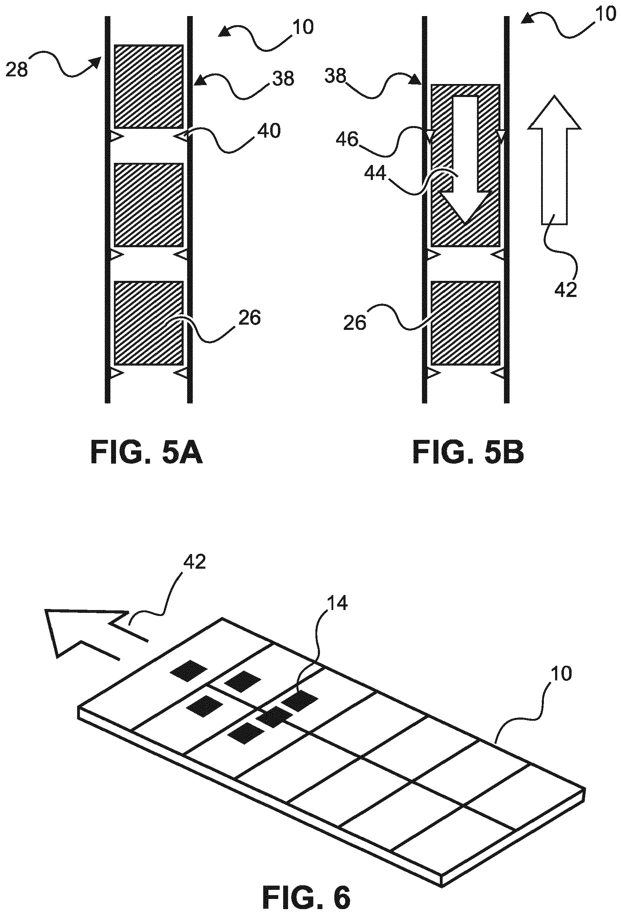

The magnetic components and/or the guiding mechanism may advantageously bring the non-magnetic metal block together automatically when they experience a high magnetic force and hence the braking force also increases. In other words, with the magnetic components and/or the guiding mechanism, the braking force as a counter force is configured to be updated with the magnetic force automatically, as will be explained in more detail in the text of the exemplary embodiments of Figs. 4 and 5.

The actuator may advantageously separate any joint non-magnetic metal blocks in order to minimize the force required to transfer the patient away from the scanner, as will be explained in more detail in the text of the exemplary embodiments of Figs. 4A and 4B.

According to an embodiment of the invention, at least one of the non-magnetic electrically conductive elements comprises a braking force controller for modulating the counter force in response to a control signal, thereby assisting with the breaking effect and/or an alignment of the patient support with respect to a bore of the MRI scanner in response to a control signal.

In other words, although the braking effect may be realized automatically in response to the magnetic field of the MRI scanner without any inputs (e.g. button) from a person, it may be advantageous to add a control signal to provide a further control of the braking force. The control signal may comprise a user input control signal, for example, to provide further manual interaction with the braking force. The user input control signal may be used for e.g. a better alignment of the patient support with respect to the bore of the MRI scanner. Alternatively or additionally, the control signal may comprise a generated control signal. The generated control signal may be obtained based on an evaluation of a position and/or an orientation of the patient support. The generated control signal may adjust the braking force and/or the alignment in response to the detected position and/or orientation of the patient support.

For example, at least one of the closed loops of conductive wire has a braking force controller in form of a feedback controller configured for modulating the counter force to automatically assist with the breaking effect and/or alignment of the patient support with respect to a bore of the MRI scanner. The feedback controller may comprise one or more software-controlled resistors. The software-controlled resistors may be configured to adjust eddy currents in response to the control signal.

For example, at least one of the non-magnetic metal blocks has a braking force controller in form of an actuator configured for joining/disjoining two or more non-magnetic metal blocks to modulate the counter force in response to the control signal.

According to an embodiment of the invention, the braking force controller is configured to control the eddy currents independently at least on two parts of the patient support, thereby modulating the counter forces at least on the two parts of the patient support independently for steering the patient support.

For example, the feedback controller is configured to control the closed loops of conductive wire independently at least on two parts (e.g. left and right sides, four corners, etc.) of the patient support, thereby modulating the counter forces at least on the two parts of the patient support independently for steering the patient support.

For example, one or more actuators are attached to the non-magnetic metal blocks and are configured to control the eddy currents independently at least on two parts (e.g. left and right sides, four corners, etc.) of the patient support.

According to an embodiment of the invention, the control signal is at least one of the following: a user input control signal, and a generated control signal based on a position and/or an orientation of the patient support detected by a position and orientation tracking device.

In an example, the braking force controller is responsive to a user input control signal for manual interaction. For example, one or more buttons may be provided for controlling an actuator under manual interaction. A user can also provide a remote control signal through a software, apps, etc. For example, software-controlled resistor may be controlled by a user, either directly or indirectly via network, for example.

In an example, the position and orientation tracing device is a camera system which monitors the position and orientation of the patient support. A control signal is then generated based on the detected position and/or orientation of the patient support, which then controls the eddy current braking system in order to automatically assist with proper braking or even alignment of the bed with respect to the bore. In an example, the position and orientation tracking device is an accelerometer or other localization device instead of camera.

This may advantageously enable assisting with the alignment of the patient support with respect to the bore of the MRI scanner.

According to an embodiment of the invention, the number of non-magnetic electrically conductive elements per unit length increases along a length of the patient support.

For example, the non-magnetic electrically conductive elements are closed loops of conductive wire. In an example, the non-magnetic electrically conductive elements are non-magnetic metal blocks. In a further example, the non-magnetic electrically conductive elements comprise both closed loops of conductive wire and non-magnetic metal blocks.

The attractive force of a magnetic material into the bore of the MRI scanner is not a constant. It is higher where the magnetic field is stronger, for example, when entering the bore. Thus, it may be advantageous to adjust the number of non-magnetic electrically conductive elements along the length of the patient support, as will be explained in more detail in the text of the exemplary embodiments of Fig. 6.

According to an embodiment of the invention, the plurality of non-magnetic electrically conductive elements is arranged in predefined positions such that the combination of the predefined positions of the non-magnetic electrically conductive elements as a brake and the magnetic field of the MRI scanner allows the guidance of the patient support to a predefined position with respect to the MRI scanner.

For example, the non-magnetic electrically conductive elements are closed loops of conductive wire. In an example, the non-magnetic electrically conductive elements are non-magnetic metal blocks. In a further example, the non-magnetic electrically conductive elements comprise both closed loops of conductive wire and non-magnetic metal blocks.

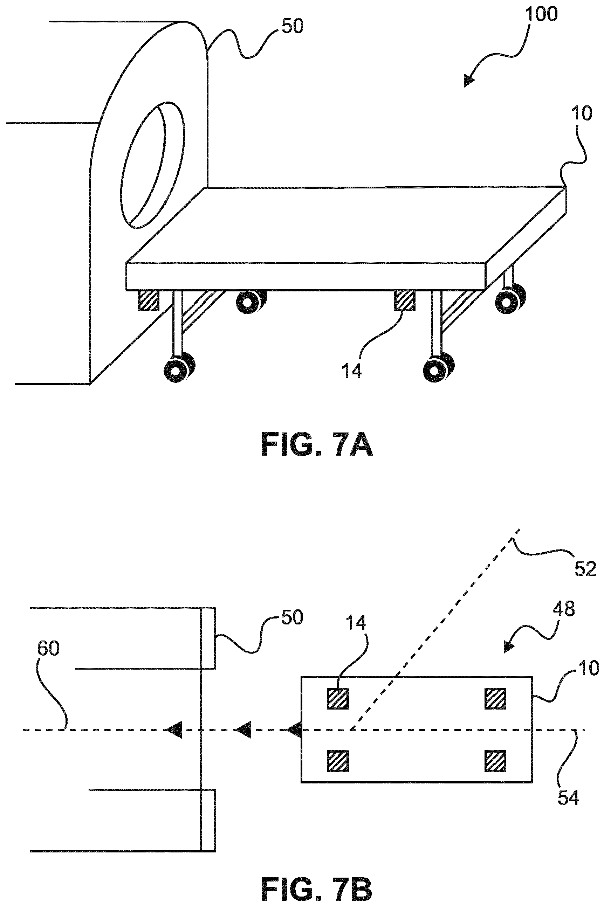

The usage of this effect may advantageously allow for a simple guiding functionality that can bring the patient support into a well predefined position e.g. to dock in an autonomous way to the scanner table and link to the patient transfer system from patient support to the scanner table, as will be explained in more detail in the text of the exemplary embodiments of Figs. 7A and 7B.

According to an embodiment of the invention, the braking device comprises an orientation guiding mechanism. Each non-magnetic electrically conductive element has a maximal cross sectional area. The orientation guiding mechanism is configured to rotate the orientation of each non-magnetic electrically conductive element into one of the following positions: the maximal cross sectional area of each non-magnetic electrically conductive element is perpendicular to a supporting plane of the patient support if the MRI scanner is a closed MRI scanner, or the maximal cross sectional area of each non-magnetic electrically conductive element is in or parallel to the supporting plane of the patient support if the MRI scanner is an open MRI scanner.

For example, the non-magnetic electrically conductive elements are closed loops of conductive wire. In an example, the non-magnetic electrically conductive elements are non-magnetic metal blocks. In a further example, the non-magnetic electrically conductive elements comprise both closed loops of conductive wire and non-magnetic metal blocks.

Braking force is only increased when the effective loop cross-section or non-magnetic metal block cross-section perpendicular to the primary magnetic field direction increases.

To function effective in the bore of an open MRI scanner, which has the primary magnetic field direction, i.e. BO-field direction, perpendicular to the patient support, it is advantageous to realize a large across section in or parallel to the supporting plane of the patient support.

To function effective in the bore of a closed MRI scanner, which has the B0-field direction along the axis of the scanner, it is advantageous to realize a large cross section perpendicular to the supporting plane of the patient support.

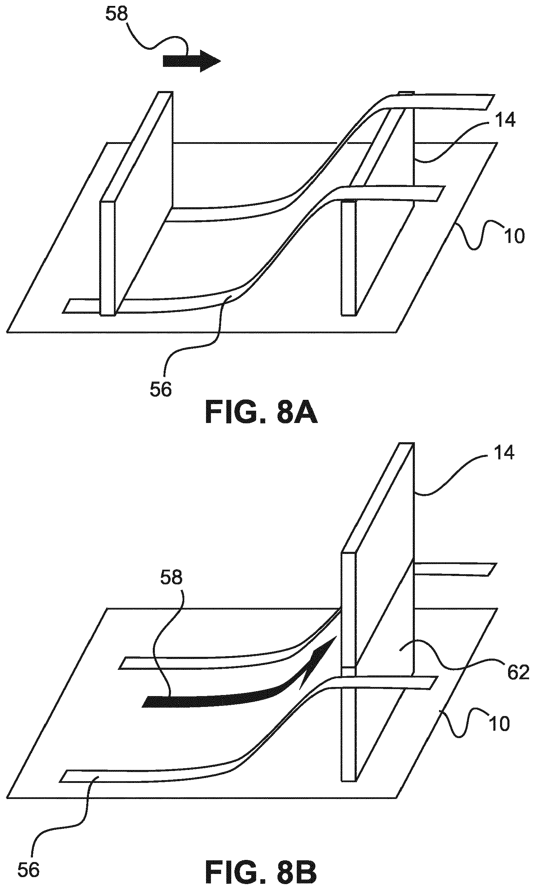

The orientation guiding mechanism may advantageously rotate the maximal cross sectional area depending upon the type of the MRI scanner, thereby creating an effective braking force for both open and closed MRI scanners, as will be explained in more detail in the text of the exemplary embodiments of Figs. 8A and 8B.

A second aspect of the invention relates to an MRI system. The MRI system comprises the patient support according to any one of the embodiments described above and below and an MRI scanner. The patient support is configured to provide a support for a patient and to facilitate a transfer of the patient in and out of the MRI scanner. The MRI scanner is configured to generate medical imaging data of the patient, as will be explained in more detail in the text of the exemplary embodiments of Figs. 7A and 7B.

An autonomous MRI system may also be part of the present invention. The autonomous MRI system comprises a patient support according to any one of the embodiments described above and below and an autonomous MRI scanner. The patient support further comprises a motor configured to drive the patient support to transfer the patient in and out of the MRI scanner and to position the patient support at a desired location for medical imaging. The autonomous MRI scanner is configured to have an MRI scan of the patient when the patient support is positioned at the desired location.

A method may also be part of the present invention for collision protection between a patient support and an MRI scanner. The method comprises the following steps: i) providing a braking device to the patient support for deaccelerating the patient support when being transferred relative to the MRI scanner, wherein the braking device comprises at least one non-magnetic electrically conductive element; and ii) inducing one or more eddy currents in response to a magnetic field of the MRI scanner to provide a counter force against an attractive force between the patient support and the MRI scanner, thereby creating a braking effect.

According to an aspect of the invention, a patient support is provided comprising one or more non-magnetic electrically conductive elements that induce eddy currents when brought into a magnetic field. The non-magnetic electrically conductive elements are modifiable by movement and/or by interruption of conductive path. The non-magnetic electrically conductive elements may be closed loops of conductive wire or non-magnetic metal blocks. The non-magnetic electrically conductive elements may comprise Aluminum or Copper.

In an example, the non-magnetic electrically conductive elements comprise closed loops of conductive wire. Each loop may be equipped with at least one user-controlled or software-controlled switch so that the eddy current effect can be substantially switched off.

In an example, the number of non-magnetic electrically conductive elements increases along the length of the patient support.

In an example, two or more non-magnetic electrically conductive elements are configured to move from electrically isolated positions to electrically contacting positions to increase the braking force.

In an example, the non-magnetic electrically conductive elements are configured to move automatically when experiencing a high magnetic force by e.g. a small magnetic component attached on the non-magnetic electrically conductive elements and/or by breakage of stoppers.

In an example, the non-magnetic electrically conductive elements are attached to actuators configured to move the non-magnetic electrically conductive elements apart to reduce the breaking force.

In an example, the combination of predefined positions of non-magnetic electrically conductive elements as a brake and a defined magnetic field of a magnet of an MRI system is configured to guide the movement of the patient support to a defined docking position.

In an example, an orientation guiding mechanism is provided and configured to rotate the non-magnetic electrically conductive elements by guiding them along a predefined path by e.g. rails, which direct the elements out of the plane of the support.

These and other aspects of the present invention will become apparent from and be elucidated with reference to the embodiments described hereinafter.

### BRIEF DESCRIPTION OF THE DRAWINGS

Exemplary embodiments of the invention will be described in the following with reference to the following drawings:
Fig. 1 shows a schematic diagram of a patient support according to an embodiment of the invention.
Fig. 2 shows a schematic diagram of a patient support according to a further embodiment of the invention.
Fig. 3A to 3C show a schematic diagram of non-magnetic electrically conductive elements according to a further embodiment of the invention.
Figs. 4A and 4B show a schematic diagram of an element joining device and an element separating device according to an embodiment of the invention.
Figs. 5A and 5B show a schematic diagram of the element joining device according to a further embodiment of the invention.
Fig. 6 shows a schematic diagram of a patient support according to a further embodiment of the invention.
Figs. 7A and 7B show a schematic diagram of a patient support according to a further embodiment of the invention in different perspectives.
Figs. 8A and 8B show a schematic diagram of a patient support according to a further embodiment of the invention in different perspectives.

### DETAILED DESCRIPTION OF THE EMBODIMENTS

Fig. 1 shows a patient support 10 for an MRI scanner according to an embodiment of the invention. The patient support 10 comprises a braking device 12 for deaccelerating the patient support when being transferred relative to the MRI scanner 50 (shown in Figs. 7A and 7B). The braking device 12 comprises at least one non-magnetic electrically conductive element 14 (shown in Figs. 2 to 8). The at least one non-magnetic electrically conductive element 14 is configured to induce one or more eddy currents in response to a magnetic field of the MRI scanner 50 to provide a counter force against an attractive force between the patient support 10 and the MRI scanner 50, thereby creating a braking effect. A patient support 10 may be e.g. a bed, a transport unit, a scan table. The patient support 10 has a supporting plane 20 on which a patient can lie. The at least one non-magnetic electrically conductive element 14 may be built into or attached to the patient support 10.

In this way, the braking forces are induced by interaction with an external magnetic field and one or more non-magnetic electrically conductive elements built into or attached to the patient support. Thus, the patient support may be deaccelerated automatically when moving in/towards an external magnetic field, i.e. the field of an MRI scanner 50. This may advantageously provide a safety feature for regular MRI scanning protocols.

In one embodiment, the braking device 12 comprises a plurality of non-magnetic electrically conductive elements 14. The plurality of non-magnetic electrically conductive elements 14 is configured and arranged to adjust the induced eddy currents in response to the magnetic field such that the counter force is adjustable against the attractive force during a transfer of the patient support 10 relative to the MRI scanner 50. Examples of the arrangements and configurations are described in Figs. 2 to 8.

Fig. 2 shows a schematic diagram of a patient support 10 according to a further embodiment of the invention. In Fig. 2, the at least one non-magnetic electrically conductive elements 14 comprises a closed loops of conductive wire 16, which may comprise Aluminum or Copper. The closed loops of conductive wire 16 may be arranged in the patient support 10 such that their effective loop cross-sections are perpendicular to a primary magnetic field direction 18, i.e. BO-field direction, to maximize the braking force. The plurality of non-magnetic electrically conductive elements 14 may also be arranged to encompass the complete cross-section of the patient support 10 to maximize the braking effect.

It is noted that the arrangement of the closed loops of conductive wires 16 in Fig. 2 is effective for the bore of a conventional closed MRI scanner 50 as here the primary magnetic field direction 18 is along a length axis of the scanner 60 (shown in Fig. 7B), i.e. in or parallel to the supporting plane 20 of the patient support 10.

In case of an open MRI scanner 50 (not shown), the closed loops of conductive wire 16 may have an effective loop cross-section parallel to the supporting plane 20 of the patient support 10, since the primary magnetic field direction of an open MRI scanner is perpendicular to the supporting plane 20 of the patient support 10.

In one embodiment, at least one of the closed loops of conductive wire 16 is provided with at least one switch 22 configured for switching the eddy currents on and off. The switch 22 comprises at least one of the following: a software controlled switch, and a user controlled switch. For example, a user may control the user controlled switch directly, for example, via a device wired to the patient support. In a further example, a user may control the user controlled switch indirectly via a software, such as wireless remote control, apps, etc. With the switch, the braking force can be switched off completely, e.g. when the patient support moves out of the bore of the MRI scanner.

In one embodiment, at least one of the non-magnetic electrically conductive elements comprises a braking force controller 24 for modulating the counter force to assist with the breaking effect and/or alignment of the patient support with respect to a bore of the MRI scanner in response to a control signal.

For example, as shown in Fig. 2, the braking force controller 24 for the closed loops of conductive wire is a feedback controller. The feedback controller may use the input from a position or acceleration sensor to automatically adjust impedances. The feedback controller may comprise one or more software-controlled resistors configured to adjust eddy currents in response to a control signal. The software-controlled resistor may have a short response time and thus may be adapted in milliseconds. The software-controlled resistors may be used to continuously modulate the braking force.

For example (not shown), the braking force controller 24 comprises one or more actuators configured for joining/disjoining two or more non-magnetic metal blocks to modulate the counter force in response to the control signal.

In one embodiment, the braking force controller 24 is configured to control the eddy currents independently at least on two parts of the patient support, thereby modulating the counter forces at least on the two parts of the patient support independently for steering the patient support.

For example, as shown in Fig. 2, the braking force controller in form of a feedback controller may be configured to control the closed loops of conductive wire independently at least on two parts (e.g. left and right sides, four corners, etc.) of the patient support, thereby modulating the counter forces at least on the two parts of the patient support independently for steering the patient support.

For example, the braking force controller in form of an actuator may be configured to join/disjoin non-magnetic metal blocks independently at least on two parts (e.g. left and right sides, four corners, etc.) of the patient support.

In one embodiment, the control signal is at least one of the following: a user input control signal, and a generated control signal based on a position and/or an orientation of the patient support detected by a position and orientation tracking device.

A user may input the control signal directly via a device wired to the patient support (e.g. a button, a touch screen, etc.), or indirectly via a network (e.g. software, apps, etc.).

The position and orientation tracking device may be a camera system or an accelerometer or other localization device for detecting the position and/or the orientation of the patient support. A control signal is then generated based on the detected position and/or orientation of the patient support, which then controls the eddy current braking system in order to automatically assist with proper braking or even alignment of the bed with respect to the bore.

In one embodiment, the feedback controller 24 is configured to control the closed loops of conductive wire 16 independently at least on two parts of the patient support 10, thereby modulating the counter forces at least on the two parts of the patient support 10 independently for steering the patient support 10. For example, as shown in Fig. 2, the feedback controller 24 is configured to control the closed loops of conductive wire 16 independently at least on the left and right sides of the patient support 10, thereby modulating the counter forces at least on the left and right sides of the patient support 10 independently for steering the patient support 10. The feedback controller 24 may be configured to control the closed loops of conductive wire 16 independently on more parts of the patient support. In an example, the feedback controller 24 is configured to control the closed loops of conductive wire 16 independently on four corners of the patient support.

This may advantageously enable assisting with the alignment of the patient support with respect to the bore of the MRI scanner.

In one embodiment, at least one of the closed loops of conductive wire 16 is configured to have low loop impedance in a passive state such that in an event of power outage the braking effect is present.

Fig. 3A to 3C show a schematic diagram of non-magnetic electrically conductive elements 14 according to a further embodiment of the invention. In Fig. 3A to 3C, the at least one non-magnetic electrically conductive elements 14 comprises a non-magnetic metal block 26, which may be made of Aluminium or Copper.

The non-magnetic metal blocks 26 may have various designs:
In Fig. 3A, the non-magnetic metal blocks 26 are in form of a set of non-magnetic metal blocks without any interruption of the area of the conducting material.

In Fig. 3B, the non-magnetic metal block 26 are in form of a comb and a rectangular block.

In Fig. 3C, the non-magnetic metal block 26 are in form of two interdigitated combs.

In one embodiment, each non-magnetic metal block 26 has a cross sectional area perpendicular to a primary magnetic field direction 18, i.e. BO-field direction, of the magnetic field. The non-magnetic metal blocks 26 are provided with an element joining device 28 configured for moving the non-magnetic metal blocks 26 from electrically isolated positions to electrically contacting positions to increase the cross sectional area perpendicular to the primary magnetic field direction 18 during a transfer of the patent support towards the magnetic field of the MRI scanner 50, thereby increasing the breaking effect. Alternatively or additionally, the non-magnetic metal blocks are provided with an element separating device 30 configured for moving the non-magnetic metal blocks from electrically contacting positions to electrically isolated positions to decrease the cross sectional area perpendicular to the primary magnetic field direction 18 during a transfer of the patient support away from the magnetic field of the MRI scanner 50, thereby decreasing the breaking effect.

In Fig. 3A, for example, in the situation that only a regular braking is required, the non-magnetic metal blocks 26 are kept separate in electrically isolated positions. In a situation where a higher braking is required, for example if the velocity or acceleration of the patient support exceeds a certain value, the non-magnetic metal blocks 26 are joined together such that the eddy currents can circulate over a larger area and the braking force increases. The more non-magnetic metal blocks 26 are joined, the higher the braking force.

A similar situation can be realized with a non-magnetic metal block comprising slots, such as the non-magnetic metal blocks in Figs. 3B and 3C. In this case, the braking force is dynamically increased by selectively closing one or more of the slots. The more slots that are closed, the higher the braking force.

It is also noted that the increased/decreased cross sectional area should be perpendicular to the primary magnetic field in order to effectively increase/decrease the braking force. Examples of the element joining device 28 and the element separating device 30 are describe in Figs. 4 and 5.

Figs. 4A and 4B show a schematic diagram of the element joining device 28 and the element separating device 30 according to an embodiment of the invention.

In Fig. 4A, the element joining device 28 comprises a plurality of magnetic components 32, each arranged on a respective non-magnetic metal block 26. Each magnetic component 32 has a dimension that is large enough to cause the attached non-magnetic metal block to move. The magnetic component 32 may have a dimension that is small enough not to cause the patient support 10 to move.

When the magnetic component 32 senses the magnetic force 34, as shown in Fig. 4B, it moves the non-magnetic metal block 26 in a direction to join a second (stationary) non-magnetic metal block. The joined non-magnetic metal blocks will produce a higher braking force, thereby retarding the motion of the entire patient support 10.

Optionally, a rail 36 may be provided to limit the degrees of freedoms of motion, which allows the non-magnetic metal blocks 26 to come closer together as they approach the MRI scanner 50 and experience the differing magnetic force as the gradients change.

With the magnetic components, as the magnetic force increases, the force on the patient support from the magnetic components also increases, thus resulting in an increasing breaking force.

Optionally, the element separating device 30 is provided, which may comprise at least one actuator. The actuator can separate any joined non-magnetic metal blocks in order to minimize the force required to transfer the patient from the MRI scanner 50.

Figs. 5A and 5B show a schematic diagram of the element joining device 28 according to a further embodiment of the invention. As an alternative concept, the element joining device 28 comprises a guiding mechanism 38 along the length of the patient support 10. The guiding mechanism 38 comprises a plurality of stoppers 40 along the guiding mechanism for keeping the non-magnetic metal blocks 26 in electrically isolated positions. The plurality of stoppers 40 is configured to allow the non-magnetic metal blocks 26 to move from electrically isolated positions to electrically contacting positions under the guidance of the guiding mechanism 38 if the attractive force exceeds a certain measure. For example, as shown in Figs. 5A and 5B, the guiding mechanism 38 is provided as rails.

As shown in Fig. 5A, the separated non-magnetic metal blocks 26 can be installed on the guiding mechanism 38 and kept in place by stoppers 40. If the patient support 10 is moved too fast towards the MRI scanner 50 in a direction 42, the induced magnetic forces 44 in the first non-magnetic metal block 26 can be strong enough to overcome the force of the stopper 40 and join with the second non-magnetic metal block 26 to form a larger block 46. If the induced magnetic forces 44 in this joined non-magnetic metal block 46 exceed the force of the second stopper 40, the non-magnetic metal block can join with the third block and so on.

The guiding mechanism 38 and the stoppers 40 may thus be used as a safety feature during manual operation, i.e. when the patient support is moved by an operator of the MRI scanner 50. Especially when moving heavy patients, it can be challenging for the operator to stop the patient support before the MRI scanner 50 so that a patient support often collides with the MRI scanner 50 itself or the patient table of the MRI scanner 50.

Fig. 6 shows a schematic diagram of a patient support 10 according to a further embodiment of the invention. The number of non-magnetic electrically conductive elements 14 per unit length increases along a length of the patient support 10. The non-magnetic electrically conductive elements 14 may be closed loops of conductive wire, non-magnetic metal blocks or a mixed of both. In this way, the braking force increases as the patient support enters the bore of the MRI scanner 50 in the direction 42, thereby countering against the increased attractive force.

Figs. 7A and 7B show a schematic diagram of a patient support 10 according to a further embodiment of the invention in different perspectives. The plurality of non-magnetic electrically conductive elements 14 (e.g. closed loops of conductive wire and/or non-magnetic metal blocks) is arranged in predefined positions 48 such that the combination of the predefined positions 48 of the non-magnetic electrically conductive elements 14 as a brake and the magnetic field of the MRI scanner 50 allows the guidance of the patient support 10 to a predefined position with respect to the MRI scanner 50. For example, as shown in Figs. 7A and 7B, the non-magnetic electrically conductive elements are arranged in predefined positions 48, e.g. on four corners of the patient support 10.

In case of a non-symmetrical movement direction 52 towards the center position of the MRI scanner 50, as shown in Fig. 7B, an unsymmetrical force due to the symmetrically mounted non-magnetic electrically conductive elements 14 appears. The force would bring the patient support 10 back into a symmetrical direct movement direction 54 to the center scanner position in case the patient support 10 is moved with a regular speed.

This may advantageously allow for a very simple guiding functionality that can bring the patient support into a well predefined position e.g. to dock in an autonomous way to the scanner table and link to the patient transfer system from patient support to scanner table. Additionally, the exact geometry and combination of the non-magnetic electrically conductive elements allow for defined trajectories and in combination with the aforementioned examples to combine and separate non-magnetic electrically conductive elements, a programmable movement direction can be realized without any external guiding structure.

Figs. 8A and 8B show a schematic diagram of a patient support 10 according to a further embodiment of the invention in different perspectives. The braking device 12 comprises an orientation guiding mechanism 56. Each non-magnetic electrically conductive element has a maximal cross sectional area 62. The orientation guiding mechanism 56 is configured to rotate the orientation of each non-magnetic electrically conductive element into one of the following positions: the maximal cross sectional area 62 of each non-magnetic electrically conductive element is perpendicular to a supporting plane 20 of the patient support if the MRI scanner is a closed MRI scanner, or the maximal cross sectional area 62 of each non-magnetic electrically conductive element is in or parallel to the supporting plane of the patient support if the MRI scanner is an open MRI scanner. The non-magnetic electrically conductive elements may be closed loops of conductive wire and/or non-magnetic metal blocks. In an example, as shown in Fig. 8, the orientation guiding mechanism is provided as rails, which direct the non-magnetic electrically conductive elements out of the plane of the patient support.

It is noted that the arrangement of non-magnetic electrically conductive element in the examples in Figs. 4 to 6 are effective for an open MRI scanner as the primary magnetic field direction is perpendicular to the supporting plane of the patient support.

To function effective in a closed MRI scanner, it is required to realize a large cross section perpendicular to the supporting plane 20 of the patient support (i.e. length axis of the scanner 60). This can be realized by using the orientation guiding mechanism 56 (e.g. rails) to rotate the orientation of the non-magnetic electrically conductive elements in the embodiments in Figs. 4 to 6 by 90 degrees such they lie perpendicular to the length axis of the scanner 60. Furthermore, the non-magnetic electrically conductive elements 14 are combined in a defined direction 58 such that their cross sectional area in the plane perpendicular to the length axis of the scanner 60 increases, as shown in Fig. 8B.

According to an embodiment of the invention, as shown in Figs.7A and 7B, an MRI system 100 is provided. The MRI system 100 comprises the patient support 10 according to any one of the embodiments described above and the MRI scanner 50. The patient support 10 is configured to provide a support for a patient and to facilitate a transfer of the patient in and out of the MRI scanner 50. The MRI scanner is configured to generate medical imaging data of the patient.

In some implementations, the MRI system may be an autonomous MRI system with the patient support and an autonomous MRI scanner. The patient support may further comprise a motor configured to drive the patient support to transfer the patient in and out of the MRI scanner and to position the patient support at a desired location for medical imaging. The autonomous MRI scanner may be configured to have an MRI scan of the patient when the patient support is positioned at the desired location.

A method may be provided for collision protection between a patient support and an MRI scanner. The method may comprise the following steps: i) providing a braking device to the patient support for deaccelerating the patient support when being transferred relative to the MRI scanner, wherein the braking device comprises at least one non-magnetic electrically conductive element; and ii) inducing one or more eddy currents in response to a magnetic field of the MRI scanner to provide a counter force against an attractive force between the patient support and the MRI scanner, thereby creating a braking effect.

It has to be noted that embodiments of the invention are described with reference to different subject matters. In particular, some embodiments are described with reference to method type claims whereas other embodiments are described with reference to the device type claims. However, a person skilled in the art will gather from the above and the following description that, unless otherwise notified, in addition to any combination of features belonging to one type of subject matter also any combination between features relating to different subject matters is considered to be disclosed with this application. However, all features can be combined providing synergetic effects that are more than the simple summation of the features.

While the invention has been illustrated and described in detail in the drawings and foregoing description, such illustration and description are to be considered illustrative or exemplary and not restrictive. The invention is not limited to the disclosed embodiments. Other variations to the disclosed embodiments can be understood and effected by those skilled in the art in practicing a claimed invention, from a study of the drawings, the disclosure, and the dependent claims.

In the claims, the word "comprising" does not exclude other elements or steps, and the indefinite article "a" or "an" does not exclude a plurality. A single processor or other unit may fulfil the functions of several items re-cited in the claims. The mere fact that certain measures are re-cited in mutually different dependent claims does not indicate that a combination of these measures cannot be used to advantage. Any reference signs in the claims should not be construed as limiting the scope.

## Claims

1. A patient support (10) for a magnetic resonance imaging (MRI) scanner, comprising:
- a braking device (12) for deaccelerating the patient support when being transferred relative to the MRI scanner;
wherein the braking device comprises at least one non-magnetic electrically conductive element (14); and
wherein the at least one non-magnetic electrically conductive element is configured to induce one or more eddy currents in response to a magnetic field of the MRI scanner to provide a counter force against an attractive force between the patient support and the MRI scanner, thereby creating a braking effect.

2. Patient support according to claim 1,
wherein the braking device comprises a plurality of non-magnetic electrically conductive elements; and
wherein the plurality of non-magnetic electrically conductive elements is configured and arranged to adjust the induced eddy currents in response to the magnetic field such that the counter force is adjustable against the attractive force during a transfer of the patient support relative to the MRI scanner.

3. Patient support according to claim 1 or 2, wherein the at least one non-magnetic electrically conductive element comprises a closed loop of conductive wire (16).

4. Patient support according to claim 3,
wherein at least one of the closed loops of conductive wire is provided with a switch (22) configured for switching the eddy currents on and off;
wherein the switch comprises at least one of the following:
- a software controlled switch; and
- a user controlled switch.

5. Patient support according to one of the claims 3 to 4,
wherein at least one of the closed loops of conductive wire is configured to have low loop impedance in a passive state such that in an event of power outage the braking effect is present.

6. Patient support according to claim 1 or 2,
wherein the at least one non-magnetic electrically conductive element comprises a non-magnetic metal block (26).

7. Patent support according to claim 6,
wherein each non-magnetic metal block has a cross sectional area perpendicular to a primary magnetic field direction (18) of the magnetic field;
wherein the non-magnetic metal blocks are provided with:
- an element joining device (28) configured for moving the non-magnetic metal blocks from electrically isolated positions to electrically contacting positions to increase the cross sectional area perpendicular to the primary magnetic field direction during a transfer of the patent support towards the magnetic field of the MRI scanner, thereby increasing the breaking effect; and/or
- an element separating device (30) configured for moving the non-magnetic metal blocks from electrically contacting positions to electrically isolated positions to decrease the cross sectional area perpendicular to the primary magnetic field direction during a transfer of the patient support away from the magnetic field of the MRI scanner, thereby decreasing the breaking effect.

8. Patient support according to claim 7,
wherein the element joining device comprises:
- a plurality of magnetic components (32), each arranged on a respective non-magnetic metal block;
wherein each magnetic component has a dimension that is large enough to cause the attached non-magnetic metal block to move; and/or
- a guiding mechanism (38) along the length of the patient support;
wherein the guiding mechanism comprises a plurality of stoppers (40) along the guiding mechanism for keeping the non-magnetic metal blocks in electrically isolated positions; and
wherein the plurality of stoppers is configured to allow the non-magnetic metal blocks to move from electrically isolated positions to electrically contacting positions under the guidance of the guiding mechanism if the attractive force exceeds a certain measure; and
wherein the element joining device the element separating device comprises at least one actuator.

9. Patient support according to one of the claims 2 to 8,
wherein at least one of the non-magnetic electrically conductive elements comprises a braking force controller (24) for modulating the counter force in response to a control signal, thereby assisting with the breaking effect and/or an alignment of the patient support with respect to a bore of the MRI scanner.

10. Patient support according to claim 9,
wherein the braking force controller is configured to control the eddy currents independently at least on two parts of the patient support, thereby modulating the counter forces at least on the two parts of the patient support independently for steering the patient support.

11. Patient support according to claim 9 or 10,
wherein the control signal is at least one of the following:
- a user input control signal; and
- a generated control signal based on a position and/or an orientation of the patient support detected by a position and orientation tracking device.

12. Patient support according to any of claims 2 to 11,
wherein the number of non-magnetic electrically conductive elements per unit length increases along a length of the patient support.

13. Patient support according to one of the claims 2 to 12,
wherein the plurality of non-magnetic electrically conductive elements is arranged in predefined positions (48) such that the combination of the predefined positions of the non-magnetic electrically conductive elements as a brake and the magnetic field of the MRI scanner allows the guidance of the patient support to a predefined position with respect to the MRI scanner.

14. Patient support according to one of the claims 2 to 13,
wherein the braking device comprises an orientation guiding mechanism (56);
wherein each non-magnetic electrically conductive element has a maximal cross sectional area (62), wherein the orientation guiding mechanism is configured to rotate the orientation of each non-magnetic electrically conductive element into one of the following positions:
- the maximal cross sectional area of each non-magnetic electrically conductive element is perpendicular to a supporting plane (20) of the patient support if the MRI scanner is a closed MRI scanner; or
- the maximal cross sectional area of each non-magnetic electrically conductive element is in or parallel to the supporting plane of the patient support if the MRI scanner is an open MRI scanner.

15. A magnetic resonance imaging (MRI) system (100), comprising:
- a patient support according to any of claims 1 to 14; and
- an MRI scanner (50);
wherein the patient support is configured to provide a support for a patient and to facilitate a transfer of the patient in and out of the MRI scanner; and
wherein the MRI scanner is configured to generate medical imaging data of the patient.
